# EUROPEAN PATENT APPLICATION

(11) **EP 3 006 940 A1**
(43) Date of publication of application: **13.04.2016**
(21) Application number: 14188456.9
(22) Date of filing: 10.10.2014
(51) Int. Cl.: G01N 33/574, C07K 14/71

(54) **Biomarkers for predicting the response to anti-angiogenic cancer therapy**

(71) Applicant: Ruprecht-Karls-Universität Heidelberg, 69117 Heidelberg (DE)
(72) Inventor: Wick, Wolfgang, Dr., 69117 Heidelberg (DE); Kessler, Tobias, 69115 Heidelberg (DE); Weiler, Markus, Dr., 69221 Dossenheim (DE)
(74) Representative: Böhmer, Thomas

(57) **Abstract**

The present invention relates to a method of predicting the response of a cancer patient to treatment with an anti-angiogenic agent, to a method of selecting a cancer patient for such treatment, and to the combined use of phosphatase and tensin homolog deleted on chromosome 10 (PTEN) and vascular endothelial growth factor receptor (VEGFR)-2 as predictive biomarkers for the response of a cancer patient to treatment with an anti-angiogenic agent. The present invention further relates to kits, the use of kits for predicting the response of a cancer patient to treatment with an anti-angiogenic agent or for selecting a cancer patient for such treatment, and to an anti-angiogenic agent for use in the treatment of cancer characterized by the presence of PTEN and the absence of VEGFR-2 in a tumor of the cancer.

SEQUENCE LISTING

<110>  Ruprecht-Karls-Universität Heidelberg

<120>  Biomarkers for predicting the response to anti-angiogenic
       cancer therapy

<130>  789-9

<160>  4

<170>  PatentIn version 3.5

<210>  1
<211>  403
<212>  PRT
<213>  Homo sapiens

<400>  1

<210>  2
<211>  1356

<212>  PRT
<213>  Homo sapiens

<400>  2

<210>  3
<211>  59
<212>  DNA
<213>  Artificial Sequence

<220>
<223>  shRNA

<400>  3
ccggctggaa tgaataccct catatctcga gatatgaggg tattcattcc agtttttg        59

<210>  4
<211>  59
<212>  DNA
<213>  Artificial Sequence

<220>
<223>  shRNA

<400>  4
aattcaaaaa accacagatc atgtggttta aactcgagtt taaaccacat gatctgtgg        59

## Description

### TECHNICAL FIELD OF THE INVENTION

The present invention relates to a method of predicting the response of a cancer patient to treatment with an anti-angiogenic agent, to a method of selecting a cancer patient for such treatment, and to the combined use of phosphatase and tensin homolog deleted on chromosome 10 (PTEN) and vascular endothelial growth factor receptor (VEGFR)-2 as predictive biomarkers for the response of a cancer patient to treatment with an anti-angiogenic agent. The present invention further relates to kits, the use of kits for predicting the response of a cancer patient to treatment with an anti-angiogenic agent or for selecting a cancer patient for such treatment, and to an anti-angiogenic agent for use in the treatment of cancer characterized by the presence of PTEN and the absence of VEGFR-2 in a tumor of the cancer.

### BACKGROUND OF THE INVENTION

Angiogenesis is a central feature associated with tumor growth of solid tumors, and thus considered to be a promising target for cancer therapy.

Agents targeting the vascular endothelial growth factor (VEGF)/VEGF receptor (VEGFR)-2 axis in cancers, such as glioblastoma, have been widely tested [1]. Phase III trials in newly diagnosed glioblastoma demonstrated a failure of the monoclonal anti-VEGF-directed antibody bevacizumab (BEV) to extend overall survival (OS), despite benefits in progression-free survival (PFS) and quality of life [2, 3]. One potential reason for the lack of an overall survival benefit is that angiogenesis inhibitors may induce tumor cell invasion into surrounding tissue in yet to be defined subgroups [4-6]. In early clinical series, a more infiltrative pattern of recurrence was observed [7], although the specificity for anti-VEGF treatments is disputed in case-control studies [8]. An analysis from the AVAglio trial also argues against a specific propensity of BEV to induce diffuse or infiltrative growth, but identified a subgroup of patients showing this tumor growth behavior [9].

Hence, the establishment of biomarkers which allow an identification of subgroups of tumors that are at increased risk to develop early resistance towards anti-angiogenic treatments and which allow the prediction of the response to anti-angiogenic treatments remains an unmet need.

It was an object of the present invention to provide methods and means for predicting the response of a cancer patient to treatment with an anti-angiogenic agent and for selecting cancer patients for treatment with an anti-angiogenic agent. It was a further object of the present invention to identify patient groups that are particularly responsive to treatment with an anti-angiogenic agent.

### SUMMARY OF THE INVENTION

In one aspect, the present invention relates to a method of predicting the response of a cancer patient to treatment with an anti-angiogenic agent, the method comprising: determining the presence or absence of
(i) phosphatase and tensin homolog deleted on chromosome 10 (PTEN), and
(ii) vascular endothelial growth factor receptor (VEGFR)-2
in a tumor sample obtained from the cancer patient.

In one embodiment, the presence of PTEN and the absence of VEGFR-2 indicate a positive response to the treatment with the anti-angiogenic agent.

In one embodiment, the positive response comprises a prolonged overall survival and/or a prolonged progression-free survival and/or a low risk to develop early evasive resistance towards treatment with an anti-angiogenic agent.

In one embodiment, the absence of PTEN and the presence of VEGFR-2 indicate a negative response to the treatment with the anti-angiogenic agent.

In one embodiment, the negative response comprises an increased risk to develop early diffuse or distant progression and/or reduced survival and/or an increased risk to develop early evasive resistance towards treatment with an anti-angiogenic agent.

In one embodiment, the cancer is glioma, preferably glioblastoma.

In one embodiment, the anti-angiogenic agent targets the vascular endothelial growth factor (VEGF) pathway.

In one embodiment, the anti-angiogenic agent is an anti-VEGF antibody, preferably bevacizumab.

In one embodiment, the presence or absence is determined on the protein level, on the DNA level and/or on the mRNA level.

In one embodiment, the presence or absence is determined on the protein level by using a technique selected from the group consisting of immunohistochemistry (IHC), Western blotting, ELISA and mass spectrometry.

In one embodiment, the presence or absence is determined on the DNA level by using a technique selected from the group consisting of quantitative PCR, genomic DNA chips, *in situ* hybridization, electrophoresis, Southern blotting and mass spectrometry.

In one embodiment, the presence or absence is determined on the mRNA level by using a technique selected from the group consisting of *in situ* hybridization, reverse transcriptase PCR, nucleic acid hybridization, electrophoresis, Northern blotting and mass spectrometry.

In one embodiment, the presence or absence is determined in a tumor infiltration zone.

In one embodiment, the method further comprises:
(i) determining the copy number of the PTEN gene and/or of the VEGFR-2 gene, and/or
(ii) determining the presence of (inactivating) mutations and/or (homozygous) deletions in the PTEN gene and/or in the VEGFR-2 gene.

In a further aspect, the present invention relates to a method of selecting a cancer patient for treatment with an anti-angiogenic agent, the method comprising:
(i) predicting the response of the cancer patient to treatment with the anti-angiogenic agent by using the method as defined above;
(ii) selecting the cancer patient for treatment with the anti-angiogenic agent if a positive response is predicted.

In one embodiment, the cancer is glioma, preferably glioblastoma.

In one embodiment, the anti-angiogenic agent targets the vascular endothelial growth factor (VEGF) pathway.

In one embodiment, the anti-angiogenic agent is an anti-VEGF antibody, preferably bevacizumab.

In a further aspect, the present invention relates to the use of phosphatase and tensin homolog deleted on chromosome 10 (PTEN) and of vascular endothelial growth factor receptor (VEGFR)-2 as predictive biomarkers for the response of a cancer patient to treatment with an anti-angiogenic agent, wherein PTEN and VEGFR-2 are used in combination.

In one embodiment, the cancer is glioma, preferably glioblastoma.

In one embodiment, the anti-angiogenic agent targets the vascular endothelial growth factor (VEGF) pathway.

In one embodiment, the anti-angiogenic agent is an anti-VEGF antibody, preferably bevacizumab.

In another aspect, the present invention relates to a kit comprising, in separate containers,
(i) means for determining the presence or absence of phosphatase and tensin homolog deleted on chromosome 10 (PTEN) in a tumor sample, preferably selected from the group consisting of PTEN-specific antibodies or antigen-binding fragments thereof and oligonucleotides comprising nucleic acids capable of hybridizing under stringent conditions to PTEN, and
(ii) means for determining the presence or absence of vascular endothelial growth factor receptor (VEGFR)-2 in a tumor sample, preferably selected from the group consisting of VEGFR-2-specific antibodies or antigen-binding fragments thereof and oligonucleotides comprising nucleic acids capable of hybridizing under stringent conditions to VEGFR-2.

In a further aspect, the present invention relates to the use of the kit as defined above for predicting the response of a cancer patient to treatment with an anti-angiogenic agent or for selecting a cancer patient for treatment with an anti-angiogenic agent.

In one embodiment, the cancer is glioma, preferably glioblastoma.

In one embodiment, the anti-angiogenic agent targets the vascular endothelial growth factor (VEGF) pathway.

In one embodiment, the anti-angiogenic agent is an anti-VEGF antibody, preferably bevacizumab.

In yet a further aspect, the present invention relates to an anti-angiogenic agent for use in the treatment of cancer, wherein the cancer is characterized by the presence of phosphatase and tensin homolog deleted on chromosome 10 (PTEN) and the absence of vascular endothelial growth factor receptor (VEGFR)-2 in a tumor, preferably in a tumor infiltration zone, of the cancer.

In one embodiment, the cancer is glioma, preferably glioblastoma.

In one embodiment, the anti-angiogenic agent targets the vascular endothelial growth factor (VEGF) pathway.

In one embodiment, the anti-angiogenic agent is an anti-VEGF antibody, preferably bevacizumab.

In a further aspect, the present invention relates to the use of an anti-angiogenic agent in the manufacture of a medicament for the treatment of cancer, wherein the cancer is characterized by the presence of phosphatase and tensin homolog deleted on chromosome 10 (PTEN) and the absence of vascular endothelial growth factor receptor (VEGFR)-2 in a tumor, preferably in a tumor infiltration zone, of the cancer.

In one embodiment, the cancer is glioma, preferably glioblastoma.

In one embodiment, the anti-angiogenic agent targets the vascular endothelial growth factor (VEGF) pathway.

In one embodiment, the anti-angiogenic agent is an anti-VEGF antibody, preferably bevacizumab.

In another aspect, the present invention relates to a method of treatment of cancer comprising administering an effective amount of an anti-angiogenic agent to a subject in need thereof, wherein the cancer is characterized by the presence of phosphatase and tensin homolog deleted on chromosome 10 (PTEN) and the absence of vascular endothelial growth factor receptor (VEGFR)-2 in a tumor, preferably in a tumor infiltration zone, of the cancer.

In one embodiment, the cancer is glioma, preferably glioblastoma.

In one embodiment, the anti-angiogenic agent targets the vascular endothelial growth factor (VEGF) pathway.

In one embodiment, the anti-angiogenic agent is an anti-VEGF antibody, preferably bevacizumab.

In yet a another aspect, the present invention relates to a method of treatment of cancer, the method comprising, as a first step, selecting a cancer patient for treatment with an anti-angiogenic agent using the method as defined above, and, as a second step, administering the anti-angiogenic agent to the cancer patient.

In one embodiment, the cancer is glioma, preferably glioblastoma.

In one embodiment, the anti-angiogenic agent targets the vascular endothelial growth factor (VEGF) pathway.

In one embodiment, the anti-angiogenic agent is an anti-VEGF antibody, preferably bevacizumab.

### DESCRIPTION OF THE FIGURES

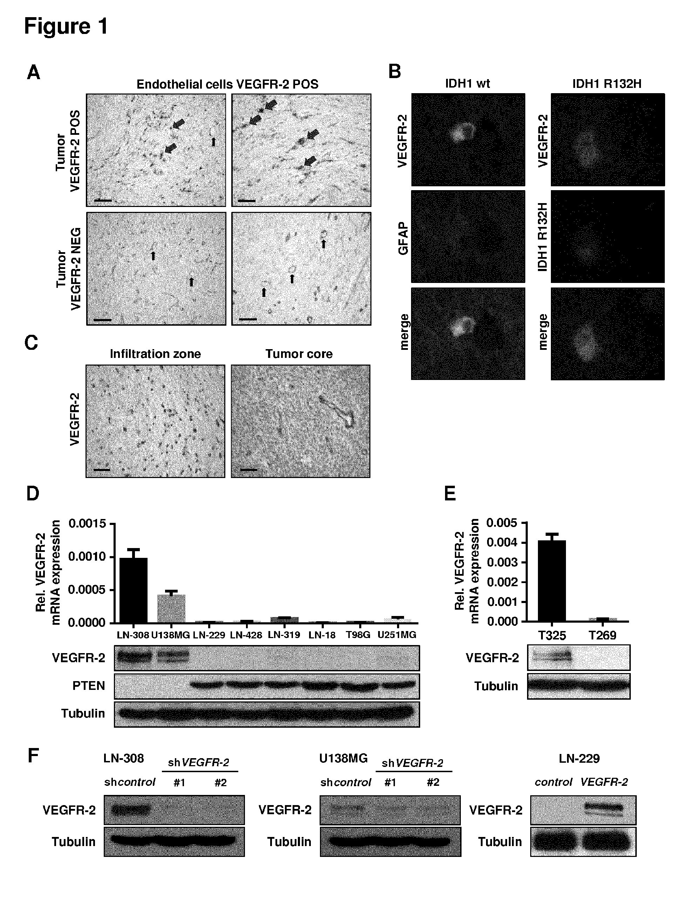

**Figure 1****.** VEGFR-2 is expressed by tumor cells in a subset of glioblastoma. **A**, Immunohistochemical analysis of *VEGFR-2* expression in primary, i.e., IDH1 wild-type glioblastoma. The upper two images show tumor tissue with VEGFR-2 expression in both endothelial and tumor cells. The lower two images depict a tumor with VEGFR-2 expression confined only to endothelial cells. VEGFR-2-positive tumor cells are indicated by big arrows, endothelial cells are marked by small arrows. Scale bars on left images, 100 µm; scale bars on right images, 50 µm. **B,** Immunofluorescence of patient-derived glioblastoma cryosections. Left column: Primary, i.e., IDH1 wild-type glioblastoma that shows positive co-immunostaining for VEGFR-2 and GFAP within the same cell proving tumor cell-specific expression of VEGFR-2. Right column: Secondary glioblastoma harboring the IDH1R132H mutation that displays positive co-immunostaining for VEGFR-2 and the mutated IDH1 protein indicating expression of VEGFR-2 in tumor cells. Scale bars, 20 µm. **C,** VEGFR-2-specific immunohistochemistry of a glioblastoma showing the infiltration zone with VEGFR-2-positive tumor cells (left) and a tumor core with VEGFR-2 expression confined to the vasculature (right). Scale bars, 20 µm. **D,** VEGFR-2 and PTEN expression in eight human glioma cell lines. Upper panel: qRT-PCR analysis, *VEGFR-2* mRNA is displayed relative to *actin* expression. Lower panel: Immunoblot analysis for VEGFR-2 and PTEN, tubulin served as a loading control. **E,** VEGFR-2 expression in two GICs. Upper panel: qRT-PCR analysis, *VEGFR-2* mRNA is displayed relative to *actin* expression. Lower panel: immunoblot analysis, tubulin served as a loading control. **F,** Immunoblot analysis of stable *VEGFR-2* knock-down *(shVEGFR-2)* and control (*shcontrol*) transfectants of LN-308 (left) and U138MG (middle) glioma cells. shRNAs targeting two different regions of *VEGFR-2* are shown. Stably VEGFR-2-overexpressing LN-229 glioma cells (right). Tubulin served as a loading control.

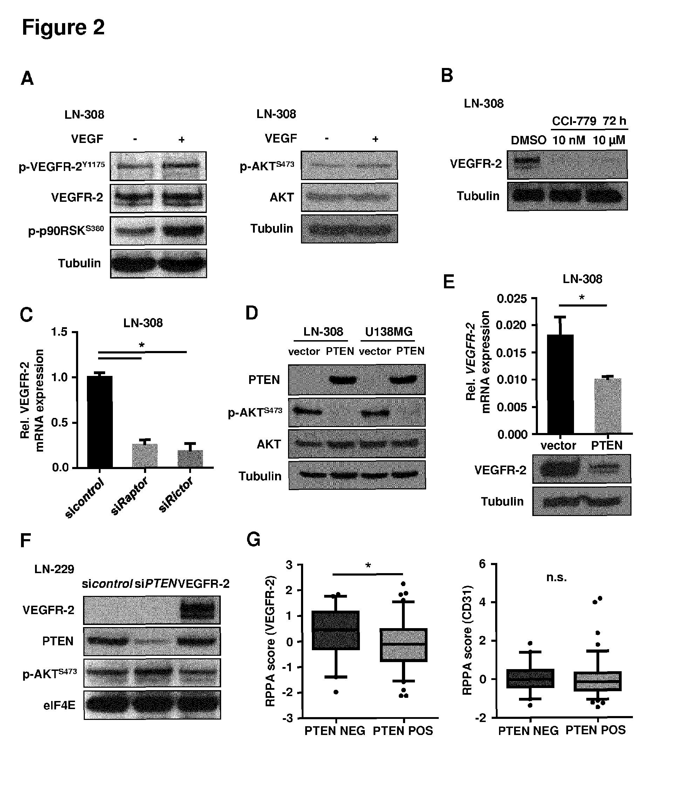

**Figure 2****.** Inhibition of the PI3K/AKT/mTOR pathway reduces tumoral VEGFR-2 expression. **A**, Immunoblot analysis of LN-308 glioma cells following stimulation with VEGF (50 ng/ml) for 5 minutes. **B,** Immunoblot analysis of lysates derived from glioma cell lines following treatment with CCI-779 at indicated concentrations. Tubulin served as a loading control. **C,** qRT-PCR analysis for *VEGFR-2* mRNA expression relative to *actin* after siRNA-mediated knock-down of *captor* (constituent of mTORCl) and *rictor* (constituent of mTORC2), respectively. **D,** Immunoblot analysis of LN-308 glioma cells with stable transfection of PTEN or empty vector control. Phospho-AKT^{S473} was used to monitor PTEN activity. Tubulin served as a loading control. **E,** qRT-PCR and immunoblot analysis for VEGFR-2 expression in PTEN-overexpressing LN-308 cells compared with controls. Tubulin served as a loading control. **F,** Immunoblot analysis of LN-229 glioma cells 72 h after transfection with *PTEN* siRNA (first two columns) or stable overexpression of VEGFR-2 (as a positive expression control; third column). Phospho-AKT^{S473} served as a control for siRNA-mediated *PTEN* inactivation, elF4E as a loading control. **G,** TCGA database analysis of glioblastoma specimens. Specimens with mutation and/or homozygous deletion of *PTEN* were denoted as 'PTEN NEG', specimens without *PTEN* mutation or homozygous deletion as 'PTEN- POS'. VEGFR-2 and CD31 reverse phase protein array (RPPA) score is shown depending on the PTEN status.

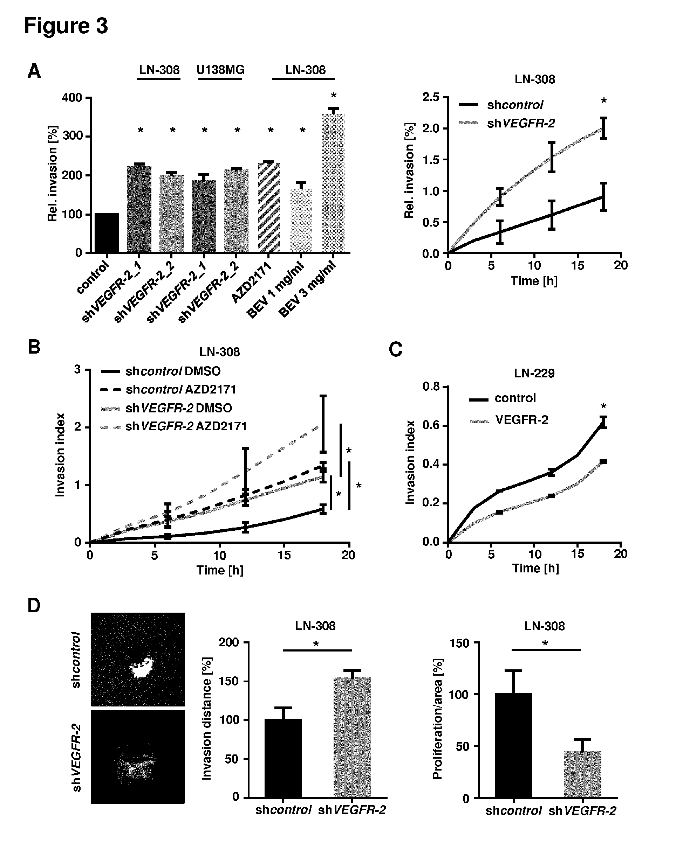

**Figure 3****.** Blockade of VEGF/VEGFR-2 signaling stimulates glioma cell invasion. **A**, Overview of glioma cell invasiveness in response to different forms of inhibition and abrogation of the VEGFR-2 pathway through *VEGFR-2* knock-down or treatment with AZD2171 or BEV. Representative results of different RTCA analyses relative to appropriate controls are shown (left). Sample of the invasive kinetic of LN-308 *shcontrol* and *shVEGFR-2* cells in a RTCA assay (right). **B,** RTCA invasion assay of LN-308 sh*control* and *shVEGFR-2* cells treated with either AZD2171 (100 nM) or equivalent DMSO. C, RTCA invasion assay of LN-229 cells stably overexpressing VEGFR-2 and respective control cells transfected with the empty expression vector. **D,** Left: Representative images of tumor invasion in the organotypic brain slice assay using beads coated with LN-308 sh*control* or *shVEGFR-2* cells. Equal GFP intensity of LN-308 sh*control* and *shVEGFR-2* cells was verified by flow cytometry before assay was started. The edge of each bead is marked by a dotted red line. Scale bars, 200 µm Middle: Invasion distance of invaded glioma cells from the edge of the bead (shown as percentage of sh*control*). Right: Proliferation of glioma cells normalized to area, representing cellular density.

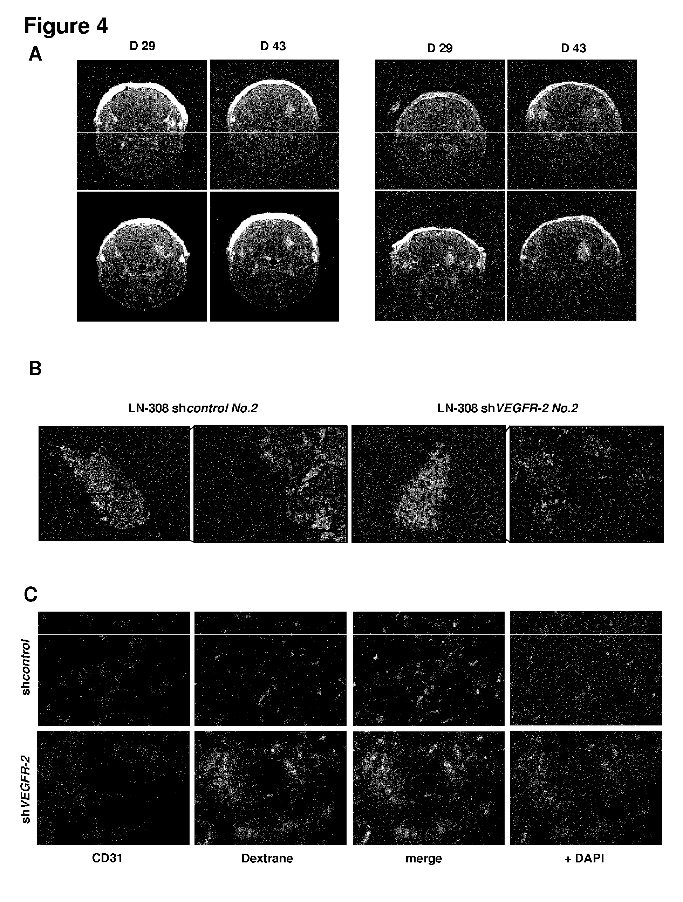

**Figure 4****.** Genetic ablation of *VEGFR-2* in glioma cells produces a more invasive growth phenotype in a xenograft mouse model. **A**, Representative T1 post contrast MRI of two mice harboring LN-308 sh*control* (left) or *shVEGFR-2* (right) tumors on days 29 and 43 after tumor cell inoculation. **B,** Immunofluorescence staining for human nestin (green) in brain slides derived from mice in (A) after termination of the experiment on day 43. Cell nuclei are enhanced with DAPI (blue). Scale bar, 100 µm. **C,** Immunofluorescence images of perfused FITC-dextran (green; vessels), CD31 (red; endothelial cells) and DAPI (blue; cell nuclei). Scale bars, 100 µm

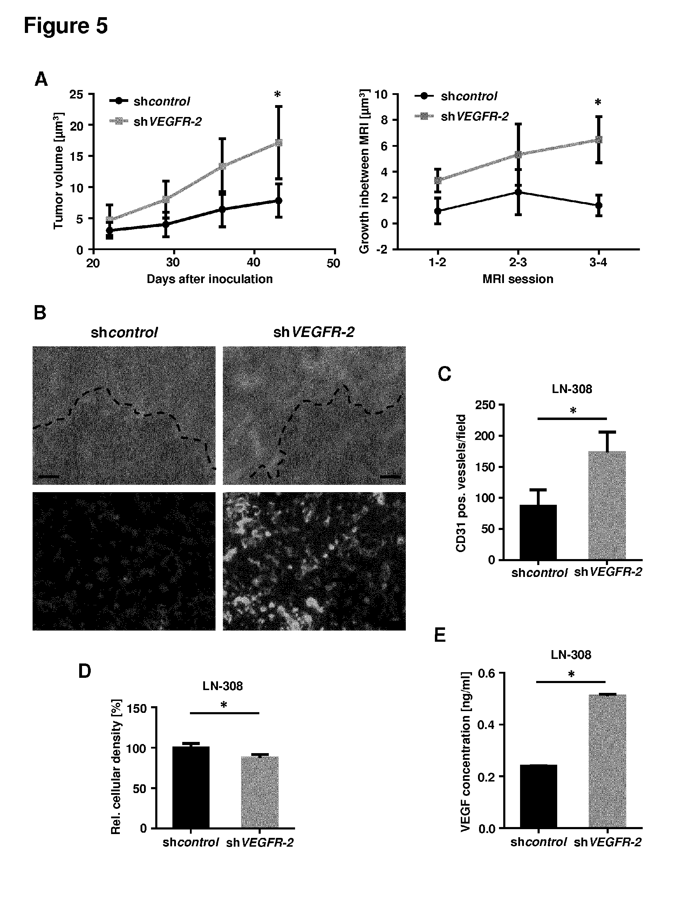

**Figure 5****.** Selective blockade of *VEGFR-2* reactively increases autocrine VEGF production and stimulates tumor vascularization. **A**, Left panel: mean tumor volume ± SD assessed in post contrast T1 MRI of mice with LN-308 sh*control* or *shVEGFR-2* tumors at indicated time points. Right panel: Tumor growth in between two MRI sessions (one week each). **B,** Upper panel: Representative images of the histological analysis of LN-308 sh*control* and *shVEGFR-2* xenografts. Hematoxylin/eosin-staining. Scale bars, 200 µm. Lower panel: *In vivo* zymography depicting the activity of MMP-2 and -9 labeled in green. Scale bars, 50 µm. **C,** vessel densities in LN-308 sh*control* and *shVEGFR-2* xenografts. Ten random microscpe fields per tumor were taken at 200-fold magnification and the amount of CD31-positive blood vessels was automatically calculated using ImageJ. **D,** ImageJ-based quantitative assessment of the cellular density in LN-308 sh*control* or *shVEGFR-2* tumors depicted in Fig. 10B. **E,** Concentrations of VEGF-A were measured by ELISA in the supernatants of LN-308 sh*control* and *shVEGFR-2* cells after 72 h of incubation.

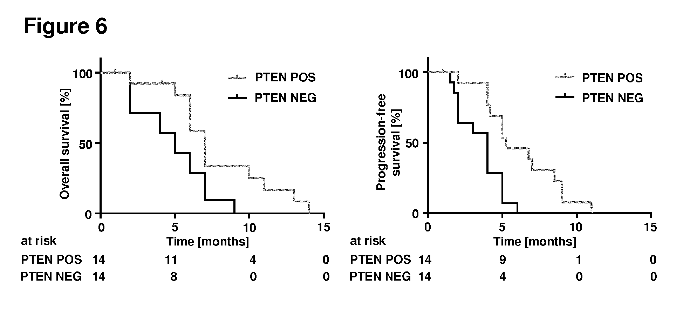

**Figure 6****.** Treatment with BEV exerts diminished anti-angiogenic but enhanced invasive effects in VEGFR-2-positive tumors. Overall survival (left) and progression-free survival (right) of patients with glioblastoma treated with BEV depending on their PTEN status. 'PTEN NEG' was defined as: i) negative PTEN immunohistochemistry or ii) positive immunohistochemistry and an inactivating mutation and/or homozygous deletion in the *PTEN* gene. Positive PTEN immunohistochemistry without mutation or deletion was evaluated 'PTEN POS'. Median overall survival: 7 *vs.* 5 months, HR 0.46, 0.13-0.67, p = 0.017. Median progression-free survival 5.2 *vs.* 4 months, HR 0.38, 0.09-0.46, *p* = 0.002.

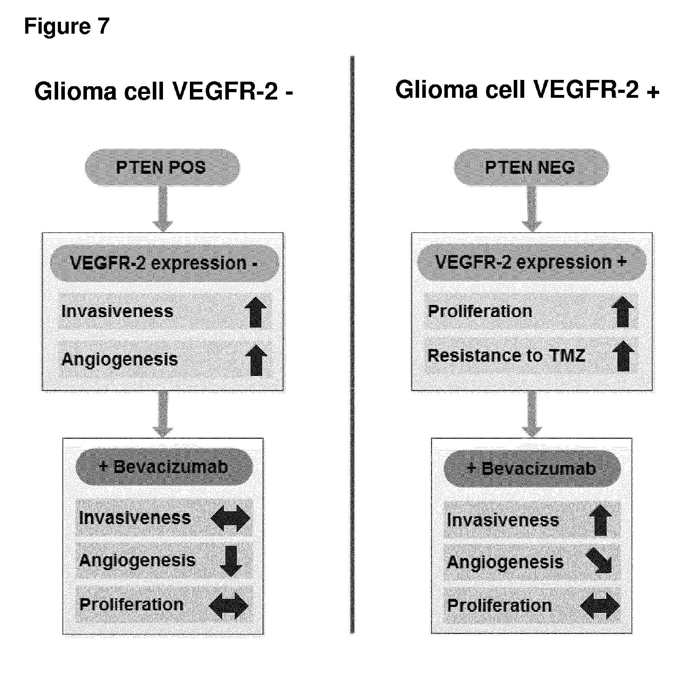

**Figure 7****.** Schematic overview summarizing cellular properties of glioblastomas with (right) or without (left) tumor cell expression of VEGFR-2 and their differential responsiveness to anti-angiogenic treatment with BEV. Of note, tumor cell expression of VEGFR-2 (right) is met only in a subgroup of PTEN-negative glioblastomas, whereas PTEN-positive tumor cells exclude tumor cell VEGFR-2 positivity.

### DETAILED DESCRIPTION OF THE INVENTION

Although the present invention is described in detail above and below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, certain elements of the present invention will be described. These elements may be listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

Phosphatase and tensin homolog deleted on chromosome 10 (PTEN), also referred to as phosphatidylinositol 3,4,5-trisphosphate 3-phosphatase and dual-specificity protein phosphatase PTEN, is a tumor suppressor protein which antagonizes the phosphoinositide 3-kinase (PI3K) signaling pathway largely by dephosphorylating phosphatidylinositol 3,4,5 trisphosphate (PIP3). Depending on the context, the term "phosphatase and tensin homolog deleted on chromosome 10 (PTEN)" or "PTEN", as used herein, may either refer to the protein itself or to a nucleic acid encoding the same, i.e. DNA or RNA (e.g., mRNA). The GenBank accession numbers for human PTEN are NM_000314 (mRNA) and NP_000305 (protein), its UniProt accession number is P60484.1. In one embodiment, PTEN has the amino acid sequence of SEQ ID NO: 1.

Vascular endothelial growth factor receptor (VEGFR)-2 is a tyrosine kinase receptor which is predominantly expressed in endothelial cells and promotes proliferation, survival, migration and differentiation of endothelial cells. Depending on the context, the term "vascular endothelial growth factor receptor (VEGFR)-2" or "VEGFR-2", as used herein, may either refer to the protein itself or to a nucleic acid encoding the same, i.e. DNA or RNA (e.g., mRNA). The GenBank accession numbers for human VEGFR-2 are NM_002253 (mRNA) and NP-002244 (protein), its UniProt accession number is P35968.2. In one embodiment, VEGFR-2 has the amino acid sequence of SEQ ID NO: 2.

Preferably, the VEGFR-2 referred to herein is VEGFR-2 expressed by tumor cells (particularly, VEGFR-2 expressed on the surface of tumor cells). Hence, in a preferred embodiment, the presence/absence of VEGFR-2 as determined in accordance with the present invention, is the presence/absence of VEGFR-2 expressed by tumor cells (particularly, VEGFR-2 expressed on the surface of tumor cells). The term "tumor cell", as used herein, refers to the cancerous cells of a tumor, and does not include non-cancerous (i.e., regular) cells, such as endothelial cells, which may also be contained in a tumor or tumor sample. In one embodiment, both the PTEN referred to herein and the VEGFR-2 referred to herein are tumor cell expressed PTEN/VEGFR-2.

As used herein, "cancer" includes a disease characterized by aberrantly regulated cellular growth, proliferation, differentiation, adhesion, and/or migration. The term "cancer" according to the invention comprises gliomas, leukemias, seminomas, melanomas, teratomas, lymphomas, neuroblastomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer and the metastases thereof. Examples thereof are lung carcinomas, mamma carcinomas, prostate carcinomas, colon carcinomas, renal cell carcinomas, cervical carcinomas, or metastases of the cancer types or tumors described above. The term cancer according to the invention also comprises cancer metastases.

In preferred embodiments of the invention, the cancer is glioma. Glioma is the most common primary tumor of the brain and arises from the supportive tissue of the brain which is formed by glial cells. There are three types of normal glial cells that can produce tumors. An astrocyte will produce astrocytomas (including glioblastomas), an oligodendrocyte will produce oligodendrogliomas, and ependymomas come from ependymal cells. Tumors that display a mixture of these different cells are called mixed gliomas (e.g., oligoastrocytomas). In addition, tumors, such as "optic nerve glioma" and "brain stem glioma" are named for their locations, not the tissue type from which they originate.

In a particularly preferred embodiment, the glioma is glioblastoma. Glioblastoma (or glioblastoma multiforme, GBM) is also known as grade IV astrocytoma. The vast majority of glioblastomas (∼90%) develop rapidly de novo in elderly patients, without clinical or histologic evidence of a less malignant precursor lesion (primary glioblastomas). Secondary glioblastomas progress from low-grade diffuse astrocytoma or anaplastic astrocytoma.

The term "tumor", as used herein, refers to all neoplastic cell growth and proliferation whether malignant or benign, and all pre-cancerous and cancerous cells and tissues. In one embodiment of the present invention, the tumor is a solid tumor. In one embodiment, the tumor is a glioma tumor or is derived from a glioma tumor (e.g. by metastasis).

The term "tumor infiltration zone", as used herein, refers to the tumor brim where single tumor cells diffusely grow and spread into the surrounding healthy organ tissue.

The term "patient", as used herein, refers to any organism such as vertebrate, particularly any mammal, including both a human and another mammal, e.g., an animal such as a rodent, a rabbit, or a monkey. The rodent may be a mouse, rat, hamster, guinea pig, or chinchilla. Preferably, the patient is a human.

The term "subject", as used herein, relates to any organism such as vertebrate, particularly any mammal, including both a human and another mammal, e.g. an animal such as a rodent, a rabbit, or a monkey. The rodent may be a mouse, rat, hamster, guinea pig, or chinchilla. Preferably, the subject is a human. In one embodiment, a subject is a subject with or suspected of having a disease, in particular cancer, also designated "patient" herein.

The term "treatment", in particular in connection with the treatment of cancer as used herein, relates to any treatment which improves the health status and/or prolongs (increases) the lifespan of a patient. Said treatment may eliminate cancer, reduce the size or the number of tumors in a patient, arrest or slow the development of cancer in a patient, inhibit or slow the development of new cancer in a patient, decrease the frequency or severity of symptoms in a patient, and/or decrease recurrences in a patient who currently has or who previously has had cancer.

"Anti-angiogenic treatment" or "anti-angiogenic therapy" refers to a treatment with an agent, e.g., a pharmacological agent, which inhibits angiogenesis. Anti-angiogenic treatment/therapy may be a monotherapy or a combined therapy with one or more anti-cancer agents, such as cytotoxic drugs and cytokines.

The term "anti-angiogenic agent", as used herein, refers to an agent which inhibits angiogenesis. Such agents are well-known to a person skilled in the art.

In a preferred embodiment, the anti-angiogenic agent targets the vascular endothelial growth factor (VEGF) pathway. The VEGF pathway involves the binding of VEGF to its receptors on the surface of endothelial cells which in turn activates intracellular tyrosine kinases, triggering multiple downstream signals that promote angiogenesis. Although there are multiple variants of both the VEGF ligand and its receptor, the angiogenic effects of this pathway are primarily mediated through the interaction of VEGF-A (the most common variant) with VEGFR-2.

In one embodiment, the anti-angiogenic agent targeting the VEGF pathway is an anti-VEGF antibody, preferably a monoclonal anti-VEGF antibody. A particularly preferred anti-VEGF antibody is bevacizumab (Avastin^{®}; BEV). Bevacizumab is a recombinant humanized monoclonal antibody that inhibits angiogenesis by binding all VEGF-A isoforms.

In another embodiment, the anti-angiogenic agent targeting the VEGF pathway is an antibody, preferably monoclonal antibody, targeting a VEGF receptor, e.g., ramucirumab and IMC-18F1.

In another embodiment, the anti-angiogenic agent targeting the VEGF pathway is a VEGF decoy receptor, e.g., aflibercept, also known as VEGF-Trap.

In yet another embodiment, the anti-angiogenic agent targeting the VEGF pathway is a small molecule inhibitor (e.g., a tyrosine kinase inhibitor, TKI), preferably selected from the group consisting of imatinib, sorafenib, sunitinib, pazopanib, axitinib, motesanib, cediranib, vatalanib, and vandetanib. The term "small molecule", as used herein, refers to a low molecular weight (< 900 Da) organic compound.

In a further embodiment, the anti-angiogenic agent targeting the VEGF pathway is a recombinant human endostatin.

The response of a cancer patient to treatment with an anti-angiogenic agent refers to a medical response characterized by objective parameters or criteria, such as objective clinical signs like the development of tumor size. The objective criteria for determining the response to anti-angiogenic therapy are well-known in the art.

A positive response to anti-angiogenic therapy preferably comprises a prolonged overall survival and/or a prolonged progression-free survival and/or a low risk to develop early evasive resistance towards treatment with an anti-angiogenic agent. In a preferred embodiment, a positive response is prolonged overall survival.

A negative response to anti-angiogenic therapy preferably comprises an increased risk to develop early diffuse or distant progression and/or reduced survival and/or an increased risk to develop early evasive resistance towards treatment with an anti-angiogenic agent.

The term "overall survival", as used herein, refers to the period of time that a person lives after being diagnosed with cancer.

The term "progression-free survival", as used herein, refers to the period of time that a person lives free from any significant increase in tumor size and number, after being diagnosed with cancer.

In one embodiment, the term "tumor sample" refers to a tumor tissue sample isolated from the cancer patient (e.g., a biopsy or resection tissue of the tumor). In a preferred embodiment, the tumor sample is a cryo-section of a tumor tissue sample or is a chemically fixed tumor tissue sample. In a more preferred embodiment, the tumor sample is a formalin-fixed and paraffin-embedded (FFPE) tumor tissue sample. In another embodiment, the tumor sample is (total) RNA or DNA extracted from the tumor tissue sample, e.g., a FFPE tumor tissue sample. Those skilled in the art are able to perform RNA or DNA extraction procedures. It is also possible to store the tumor sample material to be used/tested in a freezer and to carry out the methods of the present invention at an appropriate point in time after thawing the respective tumor sample material. The tumor sample may be obtained from the cancer patient prior to initiation of a therapeutic treatment, during the therapeutic treatment, and/or after the therapeutic treatment, i.e. prior to, during or following the administration of cancer therapy.

In a particularly preferred embodiment, the presence/absence of PTEN and VEGFR-2 is determined on the protein level, preferably by immunohistochemistry (IHC).

In one embodiment, the presence/absence of PTEN and VEGFR-2, as determined in the methods of the present invention, refers to the presence/absence of functional PTEN protein and functional VEGFR-2 protein, respectively.

In one embodiment, functional PTEN/VEGFR-2 protein is determined to be absent if i) the PTEN/VEGFR-2 immunohistochemistry is negative or ii) the PTEN/VEGFR-2 immunohistochemistry is positive and an inactivating mutation and/or homozygous deletion in the PTEN/VEGFR-2 gene is detected. In one embodiment, functional PTEN/VEGFR-2 protein is determined to be present if the PTEN/VEGFR-2 immunohistochemistry is positive and no inactivating mutation and/or homozygous deletion in the PTEN/VEGFR-2 gene is detected.

The term "biomarker", as used herein, refers to a distinctive biological or biologically derived indicator of a process, event or condition.

The term "predictive biomarker", as used herein, refers to a biomarker that can be used prior to therapy to estimate response and/or survival of a patient upon a specific treatment.

The expression "wherein PTEN and VEGFR-2 are used in combination", as used herein, refers to the fact that both the presence/absence of PTEN and the presence/absence of VEGFR-2 are determined in the tumor sample obtained from the cancer patient, which can be done either simultaneously or sequentially. The results obtained for PTEN and VEGFR-2 are then combined in order to predict the response of the cancer patient to treatment with an anti-angiogenic agent.

Preferably, the methods for predicting the response of a cancer patient to treatment with an anti-angiogenic agent or for selecting a cancer patient for treatment with an anti-angiogenic agent according to the present invention are carried out *in vitro* or *ex vivo.*

As used herein, the term "kit of parts (in short: kit)" refers to an article of manufacture comprising one or more containers and, optionally, a data carrier. Said one or more containers may be filled with one or more of the above mentioned means or reagents. Additional containers may be included in the kit that contain, e.g., diluents, buffers and further reagents, such as dNTPs or one or more controls, e.g., antibodies and/or oligonucleotides that are specific for a household gene or gene product. Said data carrier may be a non-electronical data carrier, e.g., a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g., an internet database, a centralized, or a decentralized database. Said data carrier may comprise instructions for the use of the kit in the methods of the invention.

Means that are suitable for determining the presence or absence of PTEN or VEGFR-2 in a tumor sample are known to a person skilled in the art.

According to the present invention, means for determining the presence or absence of PTEN in a tumor sample are preferably selected from the group consisting of PTEN-specific antibodies or antigen-binding fragments thereof and oligonucleotides comprising nucleic acids capable of hybridizing under stringent conditions to PTEN, and means for determining the presence or absence of VEGFR-2 in a tumor sample are preferably selected from the group consisting of VEGFR-2-specific antibodies or antigen-binding fragments thereof and oligonucleotides comprising nucleic acids capable of hybridizing under stringent conditions to VEGFR-2.

The term "antibody or antigen-binding fragment thereof", as used herein, refers to immunoglobulin molecules and immunologically active portions of immunoglobulin molecules, i.e. molecules that contain an antigen-binding site that immunospecifically binds an antigen (here: PTEN or VEGFR-2). The term "antibody" also includes all recombinant forms of antibodies, e.g. antibodies expressed in prokaryotes and unglycosylated antibodies. PTEN-specific antibodies and VEGFR-2 antibodies are commercially available (see, for example, antibodies listed in **Table 1a).**

Oligonucleotides comprising nucleic acids capable of hybridizing under stringent conditions to PTEN or VEGFR-2 may be primers or probes that can be used, for example, in quantitative PCR assays. Preferably, the oligonucleotides have a length of 9 to 60 nucleotides, more preferably 9 to 50 nucleotides, even more preferably 9 to 40 nucleotides. They may further comprise one or more detectable labels, such as fluorescent labels.

A nucleic acid is according to the invention preferably deoxyribonucleic acid (DNA) or ribonucleic acid (RNA). Nucleic acids include according to the invention genomic DNA, cDNA, mRNA, recombinantly prepared and chemically synthesized molecules. A nucleic acid may according to the invention be in the form of a single-stranded or double-stranded and linear or covalently circularly closed molecule.

"Stringent hybridization conditions", as defined herein, involve hybridizing at 68°C in 5x SSC/5xDenhardt's solution/1.0% SDS, and washing in 0.2x SSC/0.1 % SDS at room temperature, or involve an art-recognized equivalent thereof (e.g., conditions in which a hybridization is carried out at 60°C in 2.5 x SSC buffer, followed by several washing steps at 37°C in a low buffer concentration, and remains stable). The parameters of salt concentration and temperature can be varied to achieve the optimal level of identity between the oligonucleotides and the target nucleic acid. Guidance regarding such conditions is available in the art, for example, by Molecular Cloning: A Laboratory Manual, 2nd Edition, J. Sambrook et al. eds., Cold Spring Harbor Laboratory Press, Cold Spring Harbor 1989, and Ausubel et al. (eds.), 1995, Current Protocols in Molecular Biology, (John Wiley and Sons, N.Y.) at Unit 2.10.

The present invention is further illustrated by the following examples which are not to be construed as limiting the scope of the invention.

### EXAMPLES

### 1. Materials and Methods

### 1.1 Cell lines

The analyses were performed with multiple human glioblastoma cell lines (LN-18, LN-229, LN-319, T98G, U251, U138MG, LN-308, LN-428) and primary glioblastoma cells (T269, T325). Regular checks for authenticity and lack of infection, e.g. by mycoplasms, were done according to the institutional guidelines at the German Cancer Research Center, which comply with the rules provided by the AACR.

For the functional experiments with VEGFR-2 modulation in the glioblastoma cells the following shRNA constructs (Sigma-Aldrich, Taufkirchen, Germany) targeting two different regions of the *VEGFR-2* transcript were used:
sh*VEGFR-2_1*: 
5′-
CCGGCTGGAATGAATACCCTCATATCTCGAGATATGAGGGTATTCATTCCAGTTTT
TTG-3′ (SEQ ID NO: 3)
sh*VEGFR-2_2*:

5′-
AATTCAAAAACCACAGATCATGTGGTTTAAACTCGAGTTTAAACCACATGATCT
GTGG-3′ (SEQ ID NO: 4).

For generating the pcDNA3.1-VEGFR-2, a pBS(KS+) vector containing cDNA encoding the N-terminal 2.5 kb of the human *VEGFR-2* coding sequence (pBS(KS+)-VEGFR-2-EZ, EZ for extracellular) was used.

A pDONR201 cloning vector containing the full-length *PTEN* sequence was purchased from the Genomics and Proteomics Core Facility (DKFZ, Heidelberg, Germany). Cloning into the pDEST-FLAGc tagged expression vector was performed using the Gateway™ technology according to standard protocols. LN-308 and U138MG cells were transfected with the PTEN expression vector or an empty vector control using Fugene HD in a ratio of 2:7. Transfections without a vector served as further negative controls. Successfully transfected cells were selected with G418 (800 µg/ml). PTEN overexpression was determined by qRT-PCR and immunoblot (Western blot).

### 1.2 PTEN sequencing and multiplex ligation-dependent probe amplification (MLPA)

DNA was extracted from FFPE material. The PCR amplification product (2 µl) was submitted to bidirectional sequencing using the BigDye Terminator v3.1 Sequencing Kit (Applied Biosystems, Foster City, USA) or DNA was processed according to the manufacturer's protocol and the SALSA MLPA probemix P225-D1 PTEN was used (MRC-Holland, Amsterdam, Netherlands).

### 1.3 Immunohistochemistry, immunofluorescence and flow cytometry

FFPE tissue of human glioblastomas was provided by the Department of Neuropathology, Institute of Pathology, Heidelberg University Hospital, Germany. Sections cut to 3 µm were processed using a Ventana BenchMark XT® immunostainer (Ventana Medical Systems, Tucson, AZ, USA). The antibodies used are listed in **Tables 1a** and **1b.**

Staining procedure included a pretreatment with cell conditioner 1 for 60 min. Pretreatment was followed by incubation with rabbit anti-human VEGFR-2 antibody (55B11, monoclonal antibody, Cell Signaling) or rabbit anti-human PTEN (Cell Signaling) at 37°C for 30 min. Incubation was followed by Ventana standard signal amplification, UltraWash, counter-staining with one drop of hematoxylin for 4 min and one drop of bluing reagent for 4 min. For visualization, the ultraView™ Universal DAB Detection Kit (Ventana Medical Systems, Tucson, AZ, USA) was used. For immunofluorescence labeling of human tumor tissues, sections underwent identical pretreatment and were incubated with goat anti-human VEGFR-2 (R&D Systems) and either mouse anti-human IDH1R132H (Dianova, Hamburg, Germany) or mouse anti-human GFAP (Dako), respectively, at 4°C over night. Slides were afterwards incubated with secondary antibodies (Alexa Fluor® 488 nm and 568 nm; both Invitrogen) for 30 min at room temperature and covered with mounting medium containing 4',6-diamidino-2-phenylindole (DAPI; Vector Laboratories, Burlingame, CA, USA).

**Table 1a. Primary antibodies (^{a} antibodies targeting human proteins).**

| **Target** | **Source** | **Dilution/Conc.** | **Application** | **Manufacturer** | **Catalog No.** |
|---|---|---|---|---|---|
| GFAP^{a} | mouse | 1:100 | IF | DakoCytomation | M0761 |
| IDH1R132^{a} | mouse | 1:20 | IHC | Dianova | DIA-H09 |
| PTEN^{a} | rabbit | 1:1000 | WB | Cell Signaling | 9559 |
| | | 1:200 | IHC | | |
| α-Tubulin^{a} | mouse | 1:5000 | WB | Sigma Aldrich | T9026 |
| VEGFR-2^{a} | rabbit | 1:1000 | WB, FC | Cell Signaling | 2479 |
| | | 1:200 | IF, IHC | | |
| VEGFR-2^{a} | goat | 0.1 µg/mL | FC | R&D Systems | AF357 |
| p-VEGFR-2^{a} (Tyr1175) | rabbit | 1:1000 | WB | Cell Signaling | 2478 |

**Table 1b. Secondary antibodies.**

| **Target** | **Source** | **Dilution** | **Application** | **Manufacturer** | **Catalog No.** |
|---|---|---|---|---|---|
| rabbit | donkey | 1:3000 | WB | GE Healthcare | NA934V |
| mouse | sheep | 1:5000 | WB | GE Healthcare | NXA931 |
| rabbit | donkey-Alexa Fluor® 488 | 1:200 | IF, FC | Invitrogen | A-21206 |
| rat | goat-Alexa Fluor® 546 | 1:500 | IF | Invitrogen | A-11081 |
| mouse | donkey-Alexa Fluor^{®} 488 | 1:500 | IF | Jackson Immuno | 715-545-155 |
| rabbit | donkey-Alexa Fluor® 647 | 1:500 | IF | Jackson Immuno | 711-605-152 |
| goat | donkey-Alexa Fluor® 488 | 1:200 | FC | Invitrogen | A-11055 |

Flow cytometry analysis was performed in a BD FACSCanto II flow cytometer (BD Biosciences, Heidelberg, Germany). Fluorescence in a total of 10,000 events per condition was detected and results were analyzed using the FACSDiva 6.1 software (BD Biosciences). *VEGFR-2 flow cytometry:* For extracellular flow cytometry, cells were dissociated with accutase, washed and stained with rabbit polyclonal anti-human VEGFR-2 (1:200, Cell Signaling, Danvers, MA, USA) or a respective isotype control. For intracellular staining, cells were fixed and permeabilized with Cytofix/Cytoperm (BD Biosciences) prior to staining with the first antibody. As secondary antibody, donkey Alexa 488 anti-rabbit antibody was used (Invitrogen, Karlsruhe, Germany). Specific fluorescence intensity (SFI) was calculated by dividing the mean VEGFR-2 fluorescence signal by the mean isotype signal. *PI flow cytometry:* After treatment as indicated, cells dissociated with trypsin were fixed in ice cold methanol, washed and stained for 1 h in propidium iodide (40 µg/ml) with RNAse (20 µg/ml). Cell cycle distribution was quantified using the Dean-Jett-Fox method.

### 1.4 Animal experiments

All animal work was approved by the governmental authorities (Karlsruhe, Germany) and was in accordance with the NIH 'Guide for the Care and Use of Laboratory Animals'. Glioma cells were stereotactically implanted into the right brain hemisphere of CD1 nu/nu mice (Charles River Laboratories, Sulzfeld, Germany) at a depth of 3 mm.

For immunofluorescence analysis of mouse brains, sections (5 µm) were treated as follows: *Nestin Staining:* Sections were fixed with ice cold methanol before staining with the primary antibody rabbit-anti human nestin (1:200, Millipore, Billerica, MA, USA) for 2.5 h at room temperature. Unspecific binding was ruled out by staining proximate slices with a rabbit isotype antibody (1:1000, Southern Biotech, Birmingham, AL, USA). Sections were incubated with a FITC-conjugated anti-rabbit antibody (1:200, Invitrogen) for 20 min before mounting with Vectashield DAPI mounting medium (Vector Laboratories). *Dextran Staining:* Animals were perfused with FITC-labeled dextran immediately before sacrification by injection into the tail vein. Brain sections were fixed in ice cold methanol for 10 min and mounted with DAPI-containing mounting medium. *CD31 Staining:* Blood vessel stainings were conducted with a rat anti-mouse CD31 antibody (BD Biosciences) followed by Alexa Fluor® 546 goat anti-rat IgG secondary antibody (Invitrogen) and DAPI counter-staining. At least ten microscopic images (Cell Observer, Zeiss) per tumor were taken and fluorescent CD31-positive vessels were automatically counted by the NIH ImageJ software.

### 1.5 Clinical case series and TCGA assessment

German law allows individual treatments of patients in an otherwise desperate medical situation. The 28 patient cases summarized were treated on an individual basis according to high institutional standards at the University Medical Center in Heidelberg, Germany. The database of The Cancer Genome Atlas (TCGA, http://cancergenome.nih.gov) was accessed on August 4, 2014.

### 1.6 Statistical analysis

Quantitative *in vitro* data are expressed as mean ± SD, as indicated. All in vitro experiments reported here represent at least three independent replications performed in triplicate. Statistical significance was assessed by two-sided Student's t-test (Excel, Microsoft, Seattle, WA, USA). The exact Fisher test was applied to correlate VEGFR-2 with PTEN expression. Univariate survival analyses were performed using the Kaplan-Meier estimator and the log-rank test. Values of p < 0.05 were considered significant and asterisked.

### 2. Results

### 2.1 A subgroup of glioblastoma exhibits tumor cell expression of VEGFR-2, predominantly in the infiltration zone

The expression pattern of VEGFR-2 was assessed in 106 patient-derived glioblastoma specimens. Endothelial cells exhibited strong immunoreactivity for VEGFR-2. Yet, in 20 of the 106 specimens (19%), VEGFR-2 expression also on tumor cells was found (Figure **1A****).** Glioma cell-specific VEGFR-2 expression was verified by co-immunostaining with either the glial fibrillary acidic protein (GFAP) in primary glioblastomas and point-mutated (R132H) IDH1 in secondary glioblastomas, respectively **(****Figure 1B****).** Moreover, subgroup analysis of 40 specimens allowing a distinct differentiation between tumor core (n = 34) and infiltration zone *(n* = 6) disclosed that VEGFR-2-positive glioblastoma cells were more frequently found in the infiltration zone. Three of the six glioblastoma specimens (50%) of which the infiltration zones were assessable showed VEGFR-2 expression only there, whereas from the other 34 tumors only two demonstrated VEGFR-2 expression in the tumor core (5.9%, *p* = 0.018, exact Fisher test; **Figure 1C****).**

### 2.2 Loss of PTEN and activated PI3K/AKT/mTOR signaling are required for expression of VEGFR-2 in glioblastoma cells

Two of eight glioma cell lines with known PTEN status expressed VEGFR-2 mRNA and protein **(****Figure 1D****; Table 2).**

**Table 2. VEGFR-2 and PTEN status in different glioblastoma cells.**

| | **VEGFR-2** | | | **PTEN** |
|---|---|---|---|---|
| **Glioma cells** | **mRNA** | **Protein** | **Status** | **Status** |
| LN-308^{a} | ++ | ++ | POS | NEG |
| U138MG^{a} | + | + | POS | NEG |
| LN-229^{a} | - | - | NEG | POS |
| LN-428^{a} | - | - | NEG | POS |
| LN-319^{a} | (+) | - | NEG | POS |
| LN-18^{a} | - | - | NEG | POS |
| T98G^{a} | - | - | NEG | POS |
| U251^{a} | (+) | - | NEG | POS |
| T325^{b} | ++ | + | POS | NEG |
| T269^{b} | - | - | NEG | POS |

Treatment of VEGFR-2-positive LN-308 glioma cells with VEGF (50 ng/ml) led to increased phosphorylation of VEGFR-2, p90RSK and AKT **(****Figure 2A**). There was expression of VEGFR-2 only in cells with deficiency of PTEN **(****Figure 1D****),** indicating a mutually exclusive expression pattern for VEGFR-2 and PTEN in glioma cell lines and GIC cultures **(Table 2).**

Due to its activation upon loss of PTEN, mTOR might govern the expression of VEGFR-2. Pharmacologic inhibition of mTOR using CCI-779 (temsirolimus) depleted VEGFR-2 protein levels in PTEN-deficient LN-308 glioma cells **(****Figure 2B****).** Moreover, selective inactivation of either mTORC1 or mTORC2 by RNAi-mediated knock-down of either *raptor* or *rictor* caused a robust decrease in *VEGFR-2* mRNA expression suggesting that both mTOR complexes are required for VEGFR-2 expression **(****Figure 2C****).** Exogenous expression of PTEN in PTEN-deficient and VEGFR-2-positive LN-308 and U138MG glioma cells **(****Figure 2D****)** led to reduced VEGFR-2 mRNA and protein levels **(****Figure 2E****).** Knock-down of *PTEN* mediated by siRNA had no effect on VEGFR-2 expression in LN-229 cells **(****Figure 2F****).**

Immunohistochemistry (IHC) for both proteins in a series of 79 glioblastoma patient tissues demonstrated that of these, 54 tumors were either positive for PTEN or tumoral VEGFR-2 expression, and 25 tumors were negative for both markers **(Table 3).** Only glioblastomas clearly showing VEGFR-2 expression in tumor cells (in addition to its expression on endothelial cells) were evaluated as VEGFR-2-positive. Importantly, not a single PTEN-positive glioblastoma containing VEGFR-2-positive tumor cells was found (p < 0.001, exact Fisher test; **Table 3),** further supporting the inventors' hypothesis that tumoral VEGFR-2 expression requires loss of PTEN.

**Table 3. Correlation between PTEN status and VEGFR-2 expression in a series of 79 human glioblastoma specimens.**

| | **PTEN POS** | **PTEN NEG** | ***p*** |
|---|---|---|---|
| Total [n] | 37 | 42 | |
| VEGFR-2 POS [*n*], (%) | 0 (0) | 17 (40) | **< 0.001** |
| VEGFR-2 NEG [*n*]*,* (%) | 37 (100) | 25 (60) | |

PTEN IHC does not necessarily reflect genetic alterations in the *PTEN* gene possibly leading to translation of the full-length but yet dysfunctional PTEN protein. Hence, the study was completed by sequencing and multiplex ligation-dependent probe amplification (MLPA) of the *PTEN* gene in 28 of the 79 glioblastoma specimens (35%). This demonstrated PTENmutations in four (by sequencing) and deletions in all 12 tumors (by MLPA) that were tested PTEN-negative on IHC. Conversely, 16 tumors assessed as PTEN-positive on IHC revealed mutations in only two cases and deletions in 12 additional cases (the latter assumingly being functional heterodeletions).

Analysis of glioblastoma tissues from The Cancer Genome Atlas (TCGA) database (11) demonstrated increased VEGFR-2 protein expression determined with reverse phase protein array in tumors with either copy number variations (CNV) or mutations in the *PTEN* gene compared with tumors without genetic alterations. Of note, CD31 expression as a marker for endothelial cells was not affected by PTEN alterations **(****Figure 2G****).**

### 2.3 Ablation of VEGFR-2 signaling in glioma cells induces a proinvasive tumor cell phenotype

Ablation of VEGF/VEGFR-2 signaling both genetically and pharmacologically using AZD2171 or BEV increased cell invasion **(****Figure 3A****)**. As AZD2171 is a multikinase inhibitor, invasion of LN-308 sh*VEGFR-2* cells as well as VEGFR-2-negative LN-229 cells following treatment with AZD2171 was additionally assessed. LN-308 *shVEGFR-2* cells responded to AZD2171 treatment with a strong increase in invasiveness that was significantly higher than the level observed with sh*control* cells treated with AZD2171 **(****Figure 3B****).** Consistent with these results, overexpression of VEGFR-2 in LN-229 cells resulted in reduced invasiveness compared with control cells **(****Figure 3C****).** Analysis of invasion using the organotypic brain slice culture system *ex vivo* corroborated these findings by demonstrating markedly increased invasiveness for LN-308 *shVEGFR-2* cells, that corresponds to the lower cellular density in tumors formed by *shVEGFR-2* cells **(****Figure 3D****).**

### 2.4 Selective knock-down of VEGFR-2 in glioma cells induces a highly invasive and proangiogenic growth pattern in a mouse xenograft model

VEGFR-2-specific functions in glioma cells were evaluated *in vivo,* and the growth pattern of xenografted LN-308 *shVEGFR-2* cells was compared with sh*control* cells in CD1 nu/nu mice (n = 6 per group). Post contrast T1 magnetic resonance imaging (MRI) was used to segment the tumor and to calculate the tumor volumes. Mice bearing a *shVEGFR-2* tumor showed larger tumor volumes in all four MRI sessions **(****Figure 4A****).** The mean tumor volumes at day 43 post injection were 7.83 ± 2.67 µm³ for sh*control* tumors and 18.1 ± 5.8 µm³ for *shVEGFR-2* tumors (*p* = 0.024). All six sh*control* tumors showed a homogeneous contrast enhancement, whereas five of the six *shVEGFR-2* tumors appeared inhomogeneous with a hypointense central lesion, diffuse tumor borders and high contrast enhancement, suggesting infiltrative growth and disruption of the blood-brain barrier **(****Figure 4A****).** *ShVEGFR-2* tumors histologically displayed a locally destructive growth pattern with marked tumor satellite formation in contrast to sh*control* tumors that all displayed a sharp tumor rim and a higher cellular density **(****Figure 4B****).** sh*VEGFR-2* tumors demonstrated a significant increase in CD31-positive blood vessels when compared with control tumors **(****Figures 4C** **and** **5C****)** and a large peritumoral edema as well as extravasation of FITC-dextran that had been perfused in a subgroup of mice **(****Figure 4C****),** indicating leakier vessels and proving a marked disruption of the blood-brain barrier in these tumors. One likely explanation for this proangiogenic reaction is that the VEGFR-2 blockade in glioma cells activates the autocrine/paracrine VEGF/VEGFR-2 signaling pathway by increasing the production of its ligand, VEGF, which could then act on the tumor endothelium.

### 2.5 Treatment with BEV exerts diminished anti-angiogenic but enhanced invasive effects in VEGFR-2-positive tumors

It was then analyzed whether BEV-induced invasiveness is restricted to VEGFR-2-expressing glioma cells. BEV treatment was tested in RTCA assays and it was found that it causes proinvasive effects specifically in VEGFR-2-positive control but not in *shVEGFR-2* glioma cells **(****Figure 5A****).** Of note, BEV-induced invasiveness of VEGFR-2-positive glioma cells was concentration-dependent as treatment with low-dosed BEV (1 mg/ml) resulted in 1.6-fold increased invasion, whereas high-dosed BEV (3 mg/ml) stimulated invasion by more than 3-fold **(****Figure 3A****).** Corresponding VEGFR-2-dependent proinvasive effects of BEV were confirmed in the brain slice culture model: interestingly, BEV treatment of brain slices with grafted *shVEGFR-2* glioma cells resulted in a slight reduction of invasiveness **(****Figure 5B****).** Aiming at integrating the observed cellular effects in one comprehensive animal model, a nude mice model using a two-photon microscope with an implanted cranial window upon inoculation of LN-308 sh*control* or *shVEGFR-2* cells for tumor cell distribution and vessel architecture before and after BEV treatment was applied. Before treatment, density of tumor-induced blood vessels in *shVEGFR-2* tumors was significantly higher than in sh*control* tumors **(****Figure 5C,D****).** Treatment with BEV for 32 days every second day (15 mg/kg bodyweight) only caused a limited anti-angiogenic effect in sh*control* tumors **(****Figure 5C****,** upper row; **Figure 5D****).** In contrast, the vascular architecture of *shVEGFR-2* tumors became heavily disrupted upon BEV treatment compared with the pretreatment situation and with control tumors irrespective of BEV treatment **(****Figure 5C****,** lower row; **Figure 5D****).** Taken together, these results demonstrate that the presence of VEGFR-2 on glioma cells both hampers anti-angiogenesis and predisposes to an adverse evasive response when BEV is applied.

### 2.6 BEV treatment prolongs survival of patients with PTEN-proficient glioblastomas

PTEN loss has not been validated as a prognostic biomarker for overall survival as confirmed by a TCGA database analysis. However, PTEN indicative of glioma cell VEGFR-2 expression may be of prognostic value selectively in patients treated with BEV. A clinical case series of twenty-eight patients with recurrent *IDH-*wildtype glioblastoma treated with BEV was retrospectively assigned into a PTEN-positive (PTEN POS) or a PTEN-negative (PTEN NEG) group as comprehensively assessed by synoptic immunohistochemical, mutational and CNV analyses, and otherwise similar characteristics **(Table 4).**

**Table 4. BEV treated patients' characteristics.**

| | **PTEN POS** | **PTEN NEG** | ***p*** |
|---|---|---|---|
| Total [*n*] | 14 | 14 | |

| **Tumor characteristics:** | | | |
|---|---|---|---|
| VEGFR-2 POS [*n*], (%) | 0 (0) | 5 (36) | 0.041 |
| VEGFR-2 NEG [*n*]*,* (%) | 14 (100) | 9 (64) | |
| IDH1 mut [*n*], (%) | 0 (0) | 0 (0) | |

| **Patient characteristics:** | | | |
|---|---|---|---|
| Mean age [years], (range) | 49.5 (33-69) | 52.5 (34-72) | 0.454 |
| Female/male [%] | 3/11 | 4/10 | 1.000 |
| Median PFS [months] | 5.25 | 4.00 | 0.002 |
| Median OS [months] | 7.00 | 5.00 | 0.017 |

After initiation of BEV therapy, PTEN positivity correlated significantly with prolonged overall survival (median 7 *vs.* 5 months, HR 0.46, 0.13-0.67, *p* = 0.017) and progression-free survival (median 5.25 *vs.* 4 months, HR 0.38, 0.09-0.46, *p* = 0.002) compared with PTEN-negative tumors **(****Figure 6****).**

### 2.7 Summary

The data presented herein suggest the existence of VEGFR-2-expressing glioblastoma cells in patient-derived tissues, preferentially in tumor infiltration zones where oxygen supplies are higher than in the perinecrotic areas of the tumor core region. This is reflected by the strong downregulation of *VEGFR-2* observed in hypoxic conditions *in vitro.*

VEGFR-2-positive glioma cells were exclusively PTEN-deficient suggesting the requirement of PTEN loss and subsequent activation of the PI3K/AKT/mTOR signaling pathway. This was corroborated by pharmacological inhibition of mTOR using CCI-779 and RNAi-mediated blockade of mTORC1 or 2 signaling, which led to a robust downregulation of VEGFR-2. Overexpression of PTEN in PTEN-deficient cell lines reduced VEGFR-2 mRNA and protein expression. This reduction was not as strong as through inhibition ofmTORCl or 2, suggesting a more complex regulation of VEGFR-2 *via* the PI3K/AKT/mTOR pathway.

RNAi-mediated or pharmacological ablation of VEGFR-2 activity increased invasiveness of glioma cells *in vitro* and resulted in a diffuse and locally destructive growth pattern *in vivo,* concomitant with a proinvasive gene expression profile. These effects suggest that VEGFR-2 has a negative impact on glioma cell invasion. The locally destructive growth pattern is reflected by a low cellular density in VEGFR-2-negative tumors suggesting higher tumor volumes on MRI than the more proliferative VEGFR-2-positive tumors. The increased invasiveness of *shVEGFR-2* cells is not readily compatible with the localization of VEGFR-2-positive cells to the infiltration zone. Those cells may arise from oxygen tensions that are higher in the infiltration zone than in the tumor core, rather than a result of selected less invasive tumor cells.

Furthermore, it was found in three independent experimental paradigms *in vitro*, *ex vivo* and *in vivo* that ablation of VEGF/VEGFR-2 signaling, genetically or with BEV treatment, causes proinvasive reactions in the presence of VEGFR-2. Although BEV treatments performed in mouse models are limited in that only human VEGF-A secreted by the xenografted glioma cells is blocked, dramatically reduced anti-angiogenic effects of BEV in VEGFR-2-positive gliomas were found, while the absence of VEGFR-2 led to a highly instable vascular system susceptible to abrogation with BEV.

The inventors identified glioma cell VEGFR-2 expression in 19% of all glioblastoma specimens tested. Notably, this percentage roughly corresponds to the recently published portion of recurrent malignant gliomas that displayed distant or diffuse recurrence on MRI scans at the time of failure of BEV-containing treatments [8]. Furthermore, the inventors conducted a retrospective analysis of clinically well-documented patients with recurrent glioblastomas treated with BEV. Patients with PTEN-negative glioblastomas had significantly shorter survival after initiation of BEV therapy compared with PTEN-positive glioblastomas. More particularly, overall and progression-free survival was longer for patients with PTEN-positive tumors, identifying PTEN as predictive biomarker for anti-VEGF treatments.

Conclusively, the inventors identified at least two separate subgroups of glioblastomas: (i) PTEN-positive tumors that do not display glioma cell VEGFR-2 positivity and (ii) PTEN-negative tumors with a markedly increased likelihood of glioma cell VEGFR-2 expression (∼40% in the present series; **Figure 7****).** The first subgroup is less proliferative than the second and is expected to be well-responsive to anti-angiogenic treatment, and at a low risk to develop early evasive resistance. The second, in contrast, represents a more aggressive subgroup of glioblastoma being more prone to develop early resistance to chemotherapy with temozolomide (TMZ) or anti-angiogenic treatment with BEV and being at increased risk to develop early diffuse or distant progression upon anti-angiogenic therapy with BEV leading to reduced survival. Hence, this latter subgroup is likely to benefit from combined treatment concepts integrating anti-angiogenic with anti-invasive mechanisms of action. Alternatively, one could argue not to treat this glioblastoma subgroup with anti-angiogenic agents at all.

### REFERENCES

1. Beal K, Abrey LE, Gutin PH. Antiangiogenic agents in the treatment of recurrent or newly diagnosed glioblastoma: analysis of single-agent and combined modality approaches. Radiat Oncol 2011;6:2.
2. Chinot OL, Wick W, Mason W, Henriksson R, Saran F, Nishikawa R, et al. Bevacizumab plus radiotherapy-temozolomide for newly diagnosed glioblastoma. N Engl J Med 2014;370:709-22.
3. Gilbert MR, Dignam JJ, Armstrong TS, Wefel JS, Blumenthal DT, Vogelbaum MA, et al. A randomized trial of bevacizumab for newly diagnosed glioblastoma. N Engl J Med 2014;370:699-708.
4. Rubenstein JL, Kim J, Ozawa T, Zhang M, Westphal M, Deen DF, et al. Anti-VEGF antibody treatment of glioblastoma prolongs survival but results in increased vascular cooption. Neoplasia 2000;2:306-14.
5. Paez-Ribes M, Allen E, Hudock J, Takeda T, Okuyama H, Vinals F, et al. Antiangiogenic therapy elicits malignant progression of tumors to increased local invasion and distant metastasis. Cancer Cell 2009;15:220-31.
6. Lu KV, Chang JP, Parachoniak CA, Pandika MM, Aghi MK, Meyronet D, et al. VEGF Inhibits Tumor Cell Invasion and Mesenchymal Transition through a MET/VEGFR2 Complex. Cancer Cell 2012;22:21-35.
7. Norden AD, Young GS, Setayesh K, Muzikansky A, Klufas R, Ross GL, et al. Bevacizumab for recurrent malignant gliomas: efficacy, toxicity, and patterns of recurrence. Neurology 2008;70:779-87.
8. Wick A, Dorner N, Schafer N, Hofer S, Heiland S, Schemmer D, et al. Bevacizumab does not increase the risk of remote relapse in malignant glioma. Ann Neurol 2011;69:586-92.
9. Wick W, Chinot O, Mason W, Henriksson R, Saran F, Nishikawa R, et al. Patterns of tumor progression in a phase 3 study of bevacizumab (Bv) plus radiotherapy (RT) plus temozolomide (T) for newly diagnosed glioblastoma (GB). J Clin Oncol 32:5s, 2014 (suppl; abstr 2051^).

## Claims

1. A method of predicting the response of a cancer patient to treatment with an anti-angiogenic agent, the method comprising: determining the presence or absence of
(i) phosphatase and tensin homolog deleted on chromosome 10 (PTEN), and
(ii) vascular endothelial growth factor receptor (VEGFR)-2
in a tumor sample obtained from the cancer patient.

2. The method according to claim 1, wherein the presence of PTEN and the absence of VEGFR-2 indicate a positive response to the treatment with the anti-angiogenic agent, wherein, preferably, the positive response comprises a prolonged overall survival and/or a prolonged progression-free survival and/or a low risk to develop early evasive resistance towards treatment with an anti-angiogenic agent.

3. The method according to claim 1, wherein the absence of PTEN and the presence of VEGFR-2 indicate a negative response to the treatment with the anti-angiogenic agent, wherein, preferably, the negative response comprises an increased risk to develop early diffuse or distant progression and/or reduced survival and/or an increased risk to develop early evasive resistance towards treatment with an anti-angiogenic agent.

4. The method according to any of the foregoing claims, wherein the cancer is glioma, preferably glioblastoma.

5. The method according to any of the foregoing claims, wherein the anti-angiogenic agent targets the vascular endothelial growth factor (VEGF) pathway, wherein, preferably, the anti-angiogenic agent is an anti-VEGF antibody, preferably bevacizumab.

6. The method according to any of the foregoing claims, wherein the presence or absence is determined on the protein level, on the DNA level and/or on the mRNA level.

7. The method according to claim 6, wherein the presence or absence is determined on the protein level by using a technique selected from the group consisting of immunohistochemistry (IHC), Western blotting, ELISA and mass spectrometry; and/or wherein the presence or absence is determined on the DNA level by using a technique selected from the group consisting of quantitative PCR, genomic DNA chips, *in situ* hybridization, electrophoresis, Southern blotting and mass spectrometry; and/or wherein the presence or absence is determined on the mRNA level by using a technique selected from the group consisting of *in situ* hybridization, reverse transcriptase PCR, nucleic acid hybridization, electrophoresis, Northern blotting and mass spectrometry

8. The method according to any of the foregoing claims, further comprising:
(i) determining the copy number of the PTEN gene and/or of the VEGFR-2 gene, and/or
(ii) determining the presence of mutations and/or deletions in the PTEN gene and/or in the VEGFR-2 gene.

9. A method of selecting a cancer patient for treatment with an anti-angiogenic agent, the method comprising:
(i) predicting the response of the cancer patient to treatment with the anti-angiogenic agent by using the method according to any of claims 1 to 8;
(ii) selecting the cancer patient for treatment with the anti-angiogenic agent if a positive response is predicted.

10. Use of phosphatase and tensin homolog deleted on chromosome 10 (PTEN) and of vascular endothelial growth factor receptor (VEGFR)-2 as predictive biomarkers for the response of a cancer patient to treatment with an anti-angiogenic agent, wherein PTEN and VEGFR-2 are used in combination.

11. A kit comprising, in separate containers,
(i) means for determining the presence or absence of phosphatase and tensin homolog deleted on chromosome 10 (PTEN) in a tumor sample, preferably selected from the group consisting of PTEN-specific antibodies or antigen-binding fragments thereof and oligonucleotides comprising nucleic acids capable of hybridizing under stringent conditions to PTEN, and
(ii) means for determining the presence or absence of vascular endothelial growth factor receptor (VEGFR)-2 in a tumor sample, preferably selected from the group consisting of VEGFR-2-specific antibodies or antigen-binding fragments thereof and oligonucleotides comprising nucleic acids capable of hybridizing under stringent conditions to VEGFR-2.

12. Use of the kit according to claim 11 for predicting the response of a cancer patient to treatment with an anti-angiogenic agent or for selecting a cancer patient for treatment with an anti-angiogenic agent.

13. An anti-angiogenic agent for use in the treatment of cancer, wherein the cancer is **characterized by** the presence of phosphatase and tensin homolog deleted on chromosome 10 (PTEN) and the absence of vascular endothelial growth factor receptor (VEGFR)-2 in a tumor, preferably in a tumor infiltration zone, of the cancer.

14. The method according to claim 9 or the use according to claim 10 or 12 or the anti-angiogenic agent for use according to claim 13, wherein the cancer is glioma, preferably glioblastoma.

15. The method according to claim 9 or 14 or the use according to claim 10, 12 or 14 or the anti-angiogenic agent for use according to claim 13 or 14, wherein the anti-angiogenic agent targets the vascular endothelial growth factor (VEGF) pathway, wherein, preferably, the anti-angiogenic agent is an anti-VEGF antibody, preferably bevacizumab.
